# EUROPEAN PATENT APPLICATION

(11) **EP 3 967 335 A1**
(43) Date of publication of application: **16.03.2022**
(21) Application number: 20195800.6
(22) Date of filing: 11.09.2020
(51) Int. Cl.: A61L 27/24, A61L 27/36, A61L 27/38

(54) **ENGINEERED DEVITALIZED CARTILAGINOUS TISSUE FOR BONE REGENERATION**

(71) Applicant: UMC Utrecht Holding B.V., 3584 CM Utrecht (NL)
(72) Inventor: GAWLITTA, Debby, 3584 CM Utrecht (NL); LONGONI, Alessia, 3584 CM Utrecht (NL); UTOMO, Lizette, 3584 CM Utrecht (NL); ROSENBERG, Antoine Johan Wilhelm Peter, 3584 CM Utrecht (NL)
(74) Representative: V.O.

(57) **Abstract**

The invention is directed to a method for providing a devitalized cartilaginous tissue that is suitable for bone regeneration, said method comprising differentiating cells into vital cartilage cells to obtain a vital cartilaginous tissue and mildly devitalizing the vital tissue comprising drying, preferably lyophilizing, to obtain the devitalized cartilage tissue construct. Further aspects of the invention include tissue and constructs obtained by this method and use of said tissue and constructs in bone regeneration.

## Description

The invention is in the field of tissue engineering and regeneration. In particular, the invention is directed to endochondral bone regeneration. Furthermore, the invention is directed to cartilaginous tissue and constructs thereof for such regeneration and to a method for the preparation of the tissue and the constructs thereof.

Via the mechanism of endochondral bone formation, which naturally takes place in the growth plate, bone tissue can be formed from a cartilage template. Conventionally, this cartilage template can be based on engineered vital cartilage or decellularized cartilage. Moreover, the cartilage can be autologous or non-autologous (e.g. allogeneic or xenogeneic).

Although autologous engineered cartilage templates, in particular vital autologous cartilage templates can show excellent bone generation, the use of such templates faces several drawbacks. Firstly, it is only possible to treat a single person with the acquired cartilage tissue. This is particularly problematic because the chondrogenic capacity is donor dependent and, in some cases, it may take about 2 months to ensure whether the isolated cells from a particular donor are usable to engineer cartilage tissue. Therefore, in practice, the autologous approach is not clinically relevant.

Alternatively to the use of autologous templates, non-autologous templates can be used. In Gawlitta et al. Frontiers in Bioengineering and Biotechnology, 8 (2020) Article 651, endochondral bone formation using constructs based on chondrogenically differentiated, non-autologous (both allogeneic and xenogeneic) mesenchymal stromal cells (MSCs) embedded in a collagen carrier is described. Although some bone formation was observed after implanting the vital constructs, a severe immune response was triggered, in particular by the xenogeneic vital constructs, rendering the approach unsuitable for clinical translation applications. Another drawback of using vital constructs is that the supply of such constructs is limited by the complex production and limited storability.

A reduced immune response is generally observed with decellularized cartilage constructs. For example, products based on allogeneic decellularized cartilage of human donors such as Tutoplast^{™} (RTI) cartilage are clinically available for cartilage regeneration (although not for bone restoration). However, also these constructs are associated with a number of drawbacks. For example, if the constructs are based on cartilage tissue isolated from donors, of which the supply is limited, the delivery times of these product can take up to a year. Moreover, when aiming at bone regeneration, the decellularized constructs (which are typically obtained after harsh chemical, enzymatical, mechanical and/or radiative treatment), perform inferior compared to vital counterparts (see for example Cunniffe et al. Acta Biomaterialia 23 (2015) 82-90 and Gawlitta et al. Tissue engineering. Part A, 21 (2015) 694-703, and references therein).

In WO2016/160717, compositions and methods for promoting vascularized bone regeneration in a subject in need thereof by using endochondral cartilage such as devitalized cartilage or devitalized hypertrophic cartilage is described. It is known that these devitalized cartilage grafts were slower to convert to bone than the vital cartilage counterpart (see also Perry et al. The FASEB Journal Volume 30, Issue S1, Experimental Biology 2016 Meeting Abstracts, April 2016 Pages 9.2-9.2)

Therefore, there remains a need for improved and/or clinically usable constructs for endochondral bone regeneration. An object of the present invention is to provide such constructs and/or constructs that address one or more of the drawbacks of the conventional constructs.

Surprisingly, the present inventors have found that particularly mildly devitalized cartilaginous tissue shows excellent bone regeneration properties and can outperform by far even living equivalent constructs. Moreover, advantageously, these constructs can be prepared by using cells such as MSCs, such that there is no dependency on cartilage tissue isolated from donors.

The engineered devitalized cartilaginous tissue according to the present invention differs from decellularized constructs such as those described hereinabove. In the art, decellularization is generally carried out using a combination of harsh treatment steps including chemical, enzymatical, mechanical and/or radiative treatment to remove cells and cellular debris. In contrast, the devitalized tissue according to the present invention comprises remnant cellular debris. However, advantageously, the cartilaginous tissue of the present invention is not vital, and can as such be easily stored for a long period. The devitalization and the method to provide the devitalized cartilaginous tissue is accordingly a particular aspect of the invention.

Accordingly, the invention is directed to a method for providing an engineered devitalized cartilaginous tissue that is suitable for bone regeneration, said method comprising the steps of:
- providing cells, preferably encapsulated in a matrix, wherein the cells are chondrogenic or are capable of giving rise to cells of the chondrogenic lineage;
- differentiating said cells into cartilage cells comprising chondrocytes, chondrocytes expressing one or more hypertrophic markers and/or hypertrophic chondrocytes to obtain a vital cartilaginous tissue;
- mildly devitalizing the vital cartilaginous tissue comprising drying such as lyophilizing, vacuum drying, or the like to obtain the engineered devitalized cartilaginous tissue.

By mildly devitalizing the vital cartilaginous tissue in accordance with the invention, the majority, if not all of the DNA, glycosaminoglycans (GAGs) and/or total protein content is retained. Accordingly, the devitalized cartilaginous tissue typically exhibits a DNA and/or total protein and/or GAG content of no less than 50%, preferably no less than 75%, most preferably no less than 90% compared to the total DNA and/or protein and/or GAG content of equivalent vital cartilaginous tissue comprising cartilage cells (*i.e.* compared to the original living tissue it is derived from). With equivalent vital cartilaginous tissue comprising cartilage cells is meant herein tissue that is prepared the same but not dried and accordingly not devitalized (also referred to as a control).

A particularly effective and efficient way to mildly devitalize the cells comprises lyophilizing (also referred to as freeze-drying). Alternatives including vacuum drying may also devitalize the constructs, but it was found that lyophilization is the mildest and preferred of these methods. By drying the vital cartilaginous tissue at a low temperature, e.g. below 0 °C, preferably below -20 °C, more preferably below -40 °C, most preferably below -80 °C, such as by lyophilization, the protein activity can also be mostly, if not essentially entirely maintained. Accordingly, the devitalized cartilaginous tissue generally exhibits an alkaline phosphatase (ALP) activity that is no less than 50%, preferably no less than 75%, most preferably no less than 90% compared to the ALP activity of equivalent vital cartilaginous tissue comprising cartilage cells. Thus, the construct of the present invention is devitalized, not decellularized. If the constructs would be decellularized, DNA content, total protein content and in particular ALP activity would be much lower than when compared to the vital equivalent constructs.

Besides devitalizing the vital cartilage construct by drying such as lyophilization, no other treatments are required. In the art, repeated freeze-thaw cycles are also known as a devitalization technique. However, this technique reduces the bioactivity of the samples and denatures proteins (see *e.g.* Bourgine et al. Proceedings of the National Academy of Sciences of the United States of America 111 (2014) 17426-17431) and is therefore not preferred. Also, repeated freeze-thaw cycles do not involve drying of the tissue. Moreover, in particular no decellularization, chemical treatment such as treatment with detergents, mechanical treatment, heating, enzymatic treatment, genetic engineering, and/or the like is required. In fact, as detailed below, such steps are preferably not carried out in the method of the invention. In the art, for instance in White et al. Acta Biomaterialia 50 (2017) 207-219, methods are described with detergents such as sodium dodecyl sulphate (SDS) or sodium deoxycholate (SDC). Such detergent solutions with mechanical agitation can be used to solubilize cell membranes and dissociate DNA and other cellular contents and ionic detergents such as SDC solubilize cell and nucleic membranes and denature proteins. However, for the present invention, such methods are not preferred. Therefore, the method of the present invention preferably does not comprise full or partial decellularization, chemical treatment such as treatment with detergents, mechanical treatment, heating, enzymatic treatment, genetic engineering, and/or the like. Surprisingly, the present inventors actually found that any further treatment, including washing with detergents such as sodium dodecyl sulfate (SDS), reduces the effectiveness of the construct. Without wishing to be bound by theory, the present inventors believe that devitalization by drying such as lyophilization is a sufficiently mild treatment to retain more biochemical cues that promote cell attachment, migration, differentiation, and/or ultimately tissue remodeling, repair and regeneration in the construct or implantation site.

Although DNA, GAG and/or total protein content and ALP activity can be retained in the devitalized cartilaginous tissue according to the invention, the metabolic activity of the constructs is typically entirely lost because it is generally essentially free from viable cells. Accordingly, the devitalized cartilaginous tissue exhibits less than 50%, preferably less than 10%, most preferably less than 5%, compared to the metabolic activity of equivalent vital tissue comprising cartilage cells. Metabolic activity can be assessed by *e.g.* bioreduction of resazurin sodium salt.

Surprisingly, the present inventors found that the devitalized cartilaginous tissue can show a higher porosity than equivalent vital tissue. Thus, after devitalization, the porosity of the tissue is increased. Typically, the tissue comprises pores, at least at its surface, having a size of more than 0.1 µm. The pores may range up to 100 µm, for example from 0.1 to 50 µm such as about 1 to 10 µm. Without wishing to be bound by theory, the inventors believe that this porosity may contribute to the observed higher bioactivity and high remodeling rate in bone regeneration of the devitalized tissue vis-à-vis the vitalized constructs. The pores may for example serve as highways within in the tissue that accelerate the bone regeneration process. The inventors further believe that the higher porosity can be a result of the devitalization step as carried out in the method according to the present invention. Irradiation for example, which has also been suggested in the art for devitalization, does not give the porosity in accordance with the present invention. Also for this reason, the devitalization of the present method is preferred over irradiation.

As detailed hereinabove, by devitalizing the cartilaginous tissue in accordance with the invention, the extracellular matrix (ECM) of the tissue is typically essentially entirely maintained. As such, the tissue can contain ECM components such as glycosaminoglycans (GAGs), collagen type II, collagen type X and/or calcium. The presence of calcium is however less required and preferred, as this would only be used for late hypertrophy, which is less effective as further detailed herein. Without wishing to be bound by theory, the inventors believe that the ECM in the tissue plays an important role in the endochondral bone regeneration process. After administration or implementation of the tissue in the subject, cells can migrate into the devitalized tissue where they will be surrounded by the ECM (and cell remnants). Here, the retention of biochemical cues promotes cell attachment, migration, differentiation, and ultimately tissue remodeling, repair and regeneration. Moreover, using the devitalized tissue in accordance with the present invention is advantageous from a translational point of view. In particular, the absence of living cells simplifies logistics and regulations around the marketing and use of ECM-derived products. Furthermore, surprisingly, there is no need to remove or denature any immunogenic components such that allogeneic tissues derived from non-immunologically matched donors can be used. In addition, the implementation of tissue according to the invention in clinical practice is easier from a logistical point of view because, in contrast to engineered living tissues, the present devitalized cartilaginous constructs can be mass-produced, easily stored and used when needed. This is particularly the case since the tissue according to the present invention can be obtained from cells. Thus, isolation of cartilage tissue from donors is therefore not required.

For the present invention, both cells that are already chondrogenic as well as cells capable of giving rise to cells of the chondrogenic lineage can be used. Thus in principle, any cell that can be differentiated into a cartilage cell can be suitably used as the cell according to the invention. Examples of chondrogenic cells that can be differentiated into the cartilage cells include chondrocytes and any chondroprogenitor cells that can for instance be isolated from osteoarthritic cartilage tissue. Differentiating the cells in accordance with the present invention means contacting the cells within a differentiation medium, wherein the cells can differentiate and be cultivated.

Cells that can give rise to or be differentiated into cells of the chondrogenic lineage include pluripotent, multipotent, bipotent or unipotent stem cells. The cells may be chosen such that they can be directly or indirectly differentiated into the cartilage cells. For example, pluripotent stem cells can first be differentiated into the mesoderm germline and then into the mesenchymal lineage. Accordingly, pluripotent stem cells such as embryonic stem cells and induced pluripotent stem cells (iPSCs), cells of the mesoderm lineage as well as cells of the mesenchymal lineage can suitably be used as cells for the present invention. Cells of the mesenchymal lineage suitable for the invention include mesenchymal stem cells and multipotent mesenchymal stromal cells (MSCs). Mesenchymal stem cells are naturally present in various tissues (*e.g*. bone marrow) and after isolation from these tissues generally referred to as multipotent mesenchymal stromal cells or mesenchymal stromal cells. Both before and after isolation, the cells are typically abbreviated as MSCs. For the present invention, the source of the multipotent mesenchymal stromal cells may be variable and any type of tissue or organ may in principle be suitable. Specific examples include multipotent mesenchymal stromal cells isolated from fat (stromal vascular fraction or adipose-derived stem cells), bone marrow, cartilage, teeth, blood, umbilical cord, placenta, Wharton's jelly, bone (skeletal stem cells) and skeletal muscle (muscle-derived stem cells or satellite cells). Combinations of cells can also be used. Advantageously, MSCs can be pre-screened and pre-selected for their potency to be differentiated into cartilage cells. iPSCs are also preferred, for their virtually unlimited supply.

The cells according to the present invention can advantageously be non-autologous. Preferably, the cells are allogeneic. This enables omission of additional intervention for cell isolation with the patient, and would reduce costs by treating several patients with cells from one donor. Further, pooling of cells derived from multiple donors can further expand the size of the patient cohort to be treated.

*In vitro* differentiation of cells and in particular of MSCs to cartilage cells is known in the art (see for example Gawlitta et al. Tissue engineering. Part A, 21 (2015) 694-703 and Longoni et al. Frontiers in Bioengineering and Biotechnology, 8 (2020) Article 651, and references therein). With cartilage cells is herein meant any cell type in the chondrogenic lineage that is in a differentiated state beyond the naive state of the stem cells. In particular, the cells in the cartilage tissue may comprise chondroblasts, chondrocytes and/or hypertrophic chondrocytes. In addition, chondrocytes expressing one, several or all hypertrophic markers (e.g. glycosaminoglycans (GAGs), collagen type II and/or collagen type X) may be present. Such cells can be regarded as chondrocytes exhibiting hypertrophic signature, character or properties. Also, cells transdifferentiating into the osteoblastic lineage can be present, including preosteoblasts, osteoblasts, osteocytes and those in the state of transdifferentiation. By differentiating the cells into the cartilage cells, ECM is also formed.

Preferably, the cells are differentiated by stimulating chondrogenesis (*i.e.* chondrogenic differentiation) without specifically stimulating hypertrophy. Without wishing to be bound by theory, the inventors believe that chondrogenically and/or hypertrophically stimulated cells can form cartilage tissue with a hypertrophic signature and hypertrophic chondrocytes, comprising particularly favorable factors to promote bone regeneration. Accordingly, transforming growth factor-B (TGF-β) superfamily members, in particular TGF-β isoforms and/or bone morphogenetic proteins (BMPs), are preferably used for differentiating the cells. Differentiating the cells into cartilage cells expressing one, more or all hypertrophic markers (*i.e.* the cartilage tissue with a hypertrophic signature) is preferred, as such the engineered devitalized cartilaginous tissue obtainable with that vital tissue according to the invention leads to particular good results in terms of bioactivity in bone regeneration. For additionally stimulating hypertrophy, 3,3',5-triiodo-L-thyronine sodium salt (T3) and β-glycerophosphate (BGP) can be (additionally) used, but this is thus less preferred. However, when chondrogenic stimulation of the cells does not result in expression of sufficient hypertrophic markers (*e.g*. collagen type X), additional hypertrophic stimulation may be preferred. For example, this is the case for chondrocytes of healthy donors. With chondrocytes of less healthy donors (for instance isolated from osteoarthritic tissue), additional hypertrophic stimulation may not be required and is preferably not carried out.

Before differentiation, the cells are preferably encapsulated in a matrix. Encapsulation before differentiation is however not required, as the devitalized tissue can also be enlarged or encapsulated using materials to form larger composites at a later stage, as described hereinbelow. Encapsulation of stem cells is known in the art and suitable construct matrices are commercially available or can be made as desired, see also the references cited hereinabove. The matrix is preferably biodegradable, meaning that it is preferably degraded after implantation in the subject's body. A specific example of a suitable matrix is collagen type I, available from Corning, New York, USA. Other collagen types (*e.g*. type II or type X) may also be suitable, as well as hydrogels such as gelatin or gelatin-based materials, fibrin and/or calcium phosphates. The matrix may be natural, semi-synthetic or fully synthetic. It may comprise hydrogels, polymers, *e.g.* polyethylene glycols (PEGs), polylactic acid (PLA) such as poly(L-lactic) acid (PLLA), polyglycolic acid (PGA), poly(lactic-co-glycolic acid) (PGLA) or polycaprolactone (PCL), or other types of implantable biomaterials and can be a blend of different types of materials, even containing more than one of each. The matrix such as the polymer can for example be electrospun into a porous matrix structure in which the cells can be embedded and differentiated. Examples of suitable natural hydrogels are those based on collagen, gelatin, fibrin, hyaluronic acid, silk fibroin, chitosan, alginate, hydrogels or materials derived from decellularized tissues, or other ECM-derived or ECM-mimicking hydrogels, or any combination or derivative thereof. Examples of suitable semi-synthetic or fully-synthetic hydrogels include those based on photocrosslinkable, chemically or enzymatically crosslinkable or otherwise modified or functionalized natural or synthetic polymers such as gelatin methacryloyl (GelMA) (see *e.g.* Yue et al. Biomaterials 73 (2015) 254-271 and Klotz et al. Trends Biotechnology 34 (2016) 394-407) or hyaluronic acid methacrylate (HAMA) or combinations thereof. Biomaterials (*i.e.* materials based on or originating from a biological source such as tissues; and materials designed for implantation) can be suitably used, also if these biomaterials have other forms than hydrogels. The preferred presence of the matrix also makes the cartilage tissue of the present invention different from natural cartilage tissue that is isolated from a donor.

The vital and/or devitalized tissue can be provided as one or more units (also referred to as modules). Prior to the devitalization step, the size of the units comprising the vital cartilaginous tissue of the invention is preferably limited in at least one direction during *in vitro* culturing and differentiation to allow good cartilage formation. If the units would be too large in all directions, diffusion limitation would hinder proper tissue formation, resulting in a necrotic core region of the unit. Typically, this size limitation of the individual vital tissue units translates to the individual devitalized tissue units. Accordingly, the devitalized cartilaginous tissue or the unit comprising this tissue is preferably maximally 3-5 mm, more preferably 2 mm such as about 1 to 2 mm, in at least one direction. In principle, the vital and devitalized tissue can be given any shape; and the size in the other directions is not necessarily limited. For example, the tissue can be given the shape of a sheet, cylinder, block, a rod or a tube that is maximally 3-5 mm (*e.g*. between 1-2 mm) thick. In another embodiment, which is preferred, the tissue has the shape of a spheroid and/or ellipsoid.

Notably, products or constructs for implantation can take on larger shapes, greatly exceeding these dimensions in all directions by compiling single devitalized units into large geometries of clinically relevant size and dimensions. A carrier can be used to join, to immobilize and/or to provide mechanical stability to a plurality of devitalized cartilaginous tissues together to form an implantable devitalized engineered cartilaginous construct. As such, bone defects that are larger than the dimension of the constructs can be treated more easily. Accordingly, a plurality of the devitalized cartilaginous tissues or units thereof can be combined with the carrier

The carrier that can be used in the present invention can be based on any type of implantable, injectable or otherwise administrable material. For example, the carrier can be a mechanically stable, implantable device that can be coated or otherwise be provided with the devitalized engineered cartilaginous tissue to promote bone growth into the device. Such devices and carriers can for example be based on metals (*e.g.* titanium), polymers, calcium phosphates and the like. Preferably, the carrier is biodegradable such that after implantation the carrier is degraded over time in the subject allowing it to be replaced by bone.

The carrier can also comprise a combination of materials and functions. It may for example comprise a mechanically stable component and a component comprising the devitalized cartilaginous tissue.

The composite carrier material may be chosen from the same material as the matrix as described-herein above.

The carrier can comprise a natural, semi-synthetic or fully synthetic material. The carrier and the construct may be fluidic, kneadable or otherwise shapeable, for example like tooth paste, a putty or clay. Additionally, the carrier may for example be a bio-ink that can be used to produce constructs with 3D printing.

Preferably the carrier comprises one or more of natural hydrogels (e.g. those based on collagen, gelatin, fibrin, hyaluronic acid, silk fibroin, chitosan, alginate, hydrogels derived from decellularized tissues, and other ECM-derived or ECM-mimicking hydrogels, or any combinations or derivatives thereof), semi-synthetic or fully synthetic hydrogels (*e.g.* those based on photocrosslinkable, chemically or enzymatically crosslinkable or otherwise modified or functionalized natural or synthetic polymers such as gelatin methacryloyl (gelMA), hyaluronic acid methacrylate (HAMA) or combinations thereof).

The carrier may also comprise or be based on polymers, preferably biodegradable polymers such as polyethylene glycol (PEGs), polylactic acid (PLA) such as poly(L-lactic) acid (PLLA), polyglycolic acid (PGA), poly(lactic-co-glycolic acid) (PGLA) and/or polycaprolactone (PCL). The polymers may be mechanically stable.

The carrier may also comprise mechanically stable and implantable materials such as metal (*e.g*. titanium), optionally biodegradable or non-biodegradable and/or calcium phosphates.

In a further aspect, the invention is directed to a method for preparing the devitalized cartilaginous tissue construct. The method comprises immobilizing the devitalized engineered cartilaginous tissue in or on the carrier. Before immobilization, the tissue may be pretreated, for example by pulverization and/or rehydration.

In general, after devitalization of the vital tissue by drying, the devitalized tissue requires rehydration for a more effective bone regeneration. It is preferred to store the devitalized tissue dry, for reasons of stability. Rehydration of the devitalized tissue can accordingly be carried out before or after immobilizing the tissue in or on the carrier. Rehydration can also be carried out *in vivo* (*i.e.* after implantation or administration) of the tissue. *In vivo* rehydration can be carried out by using blood and/or other bodily liquids of the subject that is treated. Passive rehydration can also be used.

In yet a further aspect, the invention is directed to a kit of parts comprising the devitalized engineered cartilaginous tissue and the carrier. Such a kit may allow the clinical practitioner to combine the cartilaginous tissue and the carrier as desired. For example, the carrier can be shaped as desired and the tissue can be applied to a specific site of the carrier where, after implantation or administration, bone regeneration is desired.

It may be appreciated that the devitalized cartilaginous tissue or devitalized cartilaginous tissue construct of the invention can be used in a medical treatment method, particularly in a medical treatment comprising tissue regeneration. Accordingly, in a further aspect, the invention is directed to a method for regenerating tissue in a vertebrate, preferably a mammal, comprising administering the devitalized cartilaginous tissue or devitalized cartilaginous tissue construct. The vertebrate may be human or equine and the implant/injection location can be any bone defect in the human body. Defects can comprise any congenital defects, developmental defects or acquired defects.

Congenital defects can be for example defects where part of the bone is missing like and comparable with cleft lip palate, like and comparable with craniofacial dysostosis, like and comparable with Treacher Collins syndrome, like and comparable with skull defects, any congenital limb defects, or scoliosis. Developmental defects are defects that can occur later in life like scoliosis, any limb deformities, like hip dysplasia, hand deformities and deformities in the face *e.g.* underdevelopment of maxilla, mandible or nose. Acquired defects can be defects of any infectious loss of bone in the human body, any traumatic loss, or loss of bone after irradiation. Acquired defects also include the mouth, due to loss of dentition, which results in atrophy of the residual maxillary and/or mandibular bone after extraction of teeth and which needs bone augmentation for dental implantology

Accordingly, in the above mentioned pathologies, the devitalized cartilage may be used for treating maxillofacial and orthopedic bone defects, such as segmental mandibular or long bone defects, non-unions, restoration of bone resection sites. In addition, the cartilaginous tissue can be used to augment the existing bone tissue, such as for example required for spinal fusions, sinus floor augmentation, alveolar augmentation of mandible or maxilla.

The inventions can be illustrated by the following non-limiting examples.

### Prenote

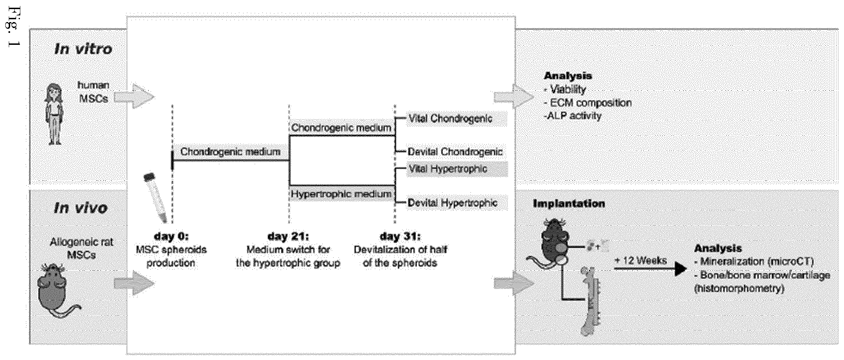

In Figure 1, the experimental outline described in Examples 1-8 below is illustrated. For the *in vitro* characterization, human MSCs were embedded in collagen hydrogels and differentiated either in chondrogenic medium (31 days) or in chondrogenic + hypertrophic media (21+10 days). After 31 days, half of the constructs of each group were devitalized. The viability of the cells and retention of ECM components was evaluated. For *in vivo* implantation, rat MSCs were used and a similar differentiation protocol was followed, albeit for the rats, bone morphogenetic protein 2 (BMP2) was also used in the medium. After 31 days, spheroids were assembled in a multi-modular construct and implanted either subcutaneously (2 spheroids per construct) or in a femur defect (8 spheroids per constructs). Carrier material control was included in the subcutaneous implantation. After 12 weeks, samples were explanted and the new bone formation was evaluated.

With respect to the statistical analyses, the following is noted. A randomized block design with Bonferroni's post-hoc correction was applied for the in vitro data to accommodate donor variation, including viability and biochemical analysis (protein, GAG, hydroxyproline and DNA content and ALP activity). A linear mixed model followed by a Bonferroni's post hoc correction was used to compare mineralization in the femur defect over time and to evaluate statistical differences in the Visipaque diffusion test (IBM SPSS 22.0, New York, USA). For the histomorphometric measures, a one-way ANOVA test was performed, followed by a Bonferroni post-hoc test (GraphPad Prism 6, San Diego, CA, USA). Differences were considered to be statistically significant for P<0.05.

### Example 1 - Isolation and expansion of human and rat bone marrow-derived MSCs

Human MSCs were isolated from bone marrow aspirates of three patients (donor 1: 20- year old, female; donor 2: 60-year old, female; donor 3: 20-year old, female) that were obtained after informed consent, in accordance to a protocol approved by the local Medical Ethics Committee (TCBio-08-001-K University Medical Center Utrecht). Ficoll-paque (GE Healthcare, Little Chalfont, UK) was used to separate the mononuclear fraction, which was further selected based on plastic adherence as previously described (Gawlitta et al., Tissue Engineering Part A 18 (2012) 1957-66). Adherent cells were cultured at 37 °C under humidified conditions and 5% carbon dioxide (CO₂) in MSC expansion medium consisting of α-MEM (22561, Invitrogen, Carlsbad, USA) supplemented with 10% heat-inactivated fetal bovine serum (51406851810, Biowest, Nuaille - France), 0.2 mM L-ascorbic acid 2-phosphate (A8960, Sigma-Aldrich, St. Louis, USA), 100U/mL penicillin with 100 mg/mL streptomycin (15140,Invitrogen), and 1 ng/mL basic fibroblast growth factor (233-FB; R&D Systems, Minneapolis, USA).

Rat MSCs were isolated from 4-week old Dark Agouti rats (Envigo, Indianapolis, USA) with the approval of the Central Authority for Scientific Procedures on Animals (CCD, no. AVD1150020172465) and the animal ethical committee of the University Medical Center Utrecht. Briefly, the rats were euthanized through CO₂ asphyxiation. After removal of the epiphysis, bone-marrow was obtained by flushing through the diaphysis with MSC expansion medium supplemented with 0.025% ethylenediaminetetraacetic acid (EDTA). Cells were allowed to adhere in a Petri dish overnight. Afterwards, StemX Vivo medium (CCM004, R&D Systems) was used for sub-culturing. Both rat and human MSCs were passaged at 80% confluency until passage 4.

### Example 2 - Chondrogenic differentiation of MSC spheroids

At passage 4, human or rat MSCs were chondrogenically differentiated. Briefly, collagen spheroids were created by encapsulating MSCs (20^{∗}10⁶/mL) in 50 µL collagen type I gel droplets (4 mg/mL) (354249, Corning, New York, USA), according to manufacturer instructions. Afterward gelation, the samples were cultured in serum-free chondrogenic medium consisting of high glucose DMEM (31966, Invitrogen) with 1% Insulin-Transferrin-Selenium (ITS) + premix (354352; Corning), 10⁻⁷ M dexamethasone (D8893; Sigma-Aldrich), 0.2 mM L-ascorbic acid 2-phosphate (A8960, Sigma-Aldrich), 100 U/mL penicillin and, 100 mg/mL streptomycin (15140, Invitrogen). To differentiate human MSCs, the medium was supplement with 10 ng/mL TGF-β1 (Peprotech, New Jersey, USA). For rat MSCs, also 100 ng/mL BMP-2 (InductOS, Wyeth/Pfizer, New York, USA) was added. Medium was refreshed daily for the first 4 days and afterwards three times per week. After 21 days of chondrogenic differentiation, half of the spheroids were subjected to hypertrophic medium, consisting of DMEM (31966, Invitrogen), 1% ITS + premix, 100 U/mL penicillin with 100 mg/mL streptomycin, 0.2 mM L-ascorbic acid-2-phosphate, 1 nM dexamethasone, 10 mM β-glycerophosphate (G9891; Sigma-Aldrich), 1 nM 3,3',5-triiodo-L-thyronine (T2877; Sigma-Aldrich). Differentiation in chondrogenic or hypertrophic medium proceeded for 10 additional days till day 31. As mentioned in the study design, human MSCs were used for the ECM characterization, whereas Dark Agouti rat MSCs were used for the in vivo experiments.

### Example 3 - Devitalization of cartilage cells and viability analyses

At 31 days, samples as prepared in Example 2 were harvested and devitalized by lyophilization (Alpha 1-2 LD, Salm en Kipp, Breukelen, the Netherlands).

Metabolic activity was assessed by bioreduction of resazurin sodium salt (R7017; Sigma-Aldrich). Briefly, the vital and devitalized chondrogenic and hypertrophic constructs were incubated for 18 hours at 37 °C in the dark with 500 µL of 10% resazurin sodium salt in chondrogenic medium without TGF-β1. Absorbance was measured on a spectrophotometer at 570nm and at 600nm for background correction (Versamax; Molecular Devices, Sunnyvale, USA). Data are presented as percentage, considering the metabolic activity of the vital chondrogenic and hypertrophic groups as 100%. The values obtained from empty collagen controls were subtracted.

To further confirm the absence of viable cells, constructs were digested using a 3 mg/mL collagenase type II (LS004177, Worthington; Lakewood, NJ, USA) in phosphate-buffered saline (PBS) digest solution for a minimum of 2 hours at 37 °C. The extracted cells were stained with 0.5 µg/mL Calcein-AM Molecular Probes, Thermo Fisher Scientific, Massachusetts, USA) for 30 minutes at 37 °C. Samples were excited at 495 nm and emission was registered at 515 nm (ASCENT Fluoroskan plate reader; Labsystem). For quantitative analysis, the signal was calibrated with known concentrations of MSCs to produce a standard curve.

Images were acquired using an Olympus IX53 inverted fluorescence microscope. Finally, the spheroid digests were re-plated in a 96-well plate and incubated with MSC expansion medium for 2 days to check for any remaining cell viability and the capacity to adhere to tissue culture plastic. Wells were washed with PBS, fixed in 10% neutral buffered formalin, and stained with methylene blue (341088-1G, Sigma-Aldrich) for 5 minutes. Images of the monolayers were taken with an Olympus IX53 inverted microscope. At least three constructs per condition for each donor were used.

### Example 4 - analysis of vital and devitalized human MSC-derived cartilage constructs

### Histological analysis

After fixation, samples as prepared in Example 2 were dehydrated in a series of increasing ethanol solutions (70-100%) and cleared in xylene. Subsequently, the samples were embedded in paraffin and sliced into 5µm thick sections (Microm HM340E; Thermo Fischer Scientific). Prior to staining, tissue sections were deparaffinized with xylene and gradually rehydrated through decreasing ethanol solutions (100-70%).

To identify cell nuclei, collagenous fibers, and glycosaminoglycans (GAGs), sections were triple stained with Weigert's hematoxylin (640490; Klinipath BV), fast green (FN1066522; Merck), and Safranin-O (FN1164048213; Merck).

To detect mineralization, von Kossa staining was performed by incubating the sections with 1% silver nitrate (209139, Sigma-Aldrich) directly under a light bulb (Philips Master TLSHO 54W 830, 1m distance), for 1 hour. The samples were subsequently washed with 5% sodium thiosulphate (A17629, Alta Aesar, Haverhill, USA) and counterstained with haematoxylin.

For collagen type 11 (0.6 µg/mL, II-116B3, Developmental Studies Hybridoma Bank) and collagen type X (10 µg/mL, 1-CO097-05, Quartett, Germany) endogenous peroxidase activity was blocked by incubating samples for 15 minutes with 0.3% H202. For collagen type II staining, antigen retrieval was done by a sequential treatment of 1 mg/mL pronase (Sigma-Aldrich) and 10 mg/mL hyaluronidase (Sigma-Aldrich) for 30 minutes each at 37 °C. For collagen type X staining, antigens were retrieved by sequential incubation with 1 mg/mL pepsin (Sigma-Aldrich) at pH 2.0 for 2 hours and 10 mg/mL hyaluronidase for 30 minutes, both at 37 °C. Prior to primary antibody incubation, samples were blocked with 5% BSA/PBS for 30 minutes at room temperature. Samples were incubated with the primary antibody overnight at 4 °C. After 30 minutes incubation with the secondary brightvision antibody (VWRKDPVM110HRP, BrightVision poly HRP-anti-mouse IgG, VWR, Radnor, USA), the labels were visualized by 3,3'-diaminobenzidine oxidation. Sections were then counterstained with haematoxylin, washed, dehydrated, and mounted with depex mounting medium. Mouse isotypes (X0931, Dako, Santa Clara, USA) were used as negative controls at the same concentration as the primary antibodies.

Images were taken with an Olympus BX51 microscope (Olympus DP73 camera, Olympus, Hamburg, Germany).

### Biochemical analysis

For total protein quantification, samples prepared according to Example 2 were digested with 0.5 mg/mL collagenase II for 5 hours at 37 °C. Protein concentration was determined using the Pierce^{™} BCA protein assay kit (23225, ThermoFisher Scientific) according to manufacturer's instructions. Known concentrations of bovine serum albumin were used to create a standard curve. Absorbance was measured at 562 nm.

Samples for GAGs and collagen analysis were digested overnight at 60 °C in papain digestion buffer (250 µg/mL papain, 0.2 M NaH₂PO₄, 0.1 M EDTA and 0.01 M DL-cysteine hydrochloride (all Sigma-Aldrich)). The total amount of GAGs was determined using the 1,9-dimethyl-methylene blue (DMMB pH 3.0; Sigma-Aldrich) assay (Farndale et al. Biochimica et Biophysica Acta 883 (1986) 173-7). Known concentration of shark chondroitin sulphate C (Sigma-Aldrich) was used as standard. Absorbance values were detected at 525 and 595 and expressed as a ratio.

To measure hydroxyproline content, 50 µL of the papain digests of all the samples were freeze-dried overnight. Afterwards, samples were hydrolyzed by sequential incubation with 0.4 M NaOH at 108 °C and 1.4 M citric acid. Hydroxyproline contents were measured using a colorimetric method (extinction 570 nm), with chloramine-T and dimethylaminobenzaldehyde as reagents as previously described (Creemers et al. BioTechniques 22 (1997) 656-658). Hydroxyproline (Merck) was used as a standard.

Alkaline phosphatase (ALP) activity was measured by using the p-Nitrophenyl Phosphate (pNPP) substrate system (N2765; Sigma). Different concentrations of ALP with a known activity (U/mL) were used as standard curve. The constructs and the standard series were incubated with the pNPP substrate at 37°C for 8 minutes. Absorbance was measured at 405nm with 655nm as a reference wavelength.

The DNA content was quantified using a Quant-iT Picogreen dsDNA assay (P11496, Thermo Fisher Scientific) according to the manufacturer's instructions. Four constructs were used per condition for each donor in all analyses. Two MSC donors were used for the total protein quantification. Three MSC donors were used for the analysis of GAGs, collagen, ALP and DNA.

### Evaluation of the ECM porosity and susceptibility to degradation

Fixed samples were dehydrated using a critical point dryer (CPD 030, Bal-Tec) for scanning electron microscopy (n=1 per group). After gold sputtering (JEOL, JFC-1300, JEOL Ltd, Tokyo, Japan), samples were imaged using a scanning electron microscope (SEM; JEOL JSM-5600, JEOL Ltd).

For the degradation study, samples (n=3 per group) were incubated with 10 U/mL collagenase II (Worthington) in plain DMEM at 37 °C. Medium was collected and replaced after 1, 2, 4, 12, 18, 24, 36, 48, 60, 72, and 80 hours. The collected medium was processed as described above to measure hydroxyproline.

To indirectly measure the porosity of the constructs, samples (n=3 per group, 1 MSC donor) were immobilized at the bottom of a custom-made mold of 3% agarose gel. The top of the spheroid was exposed to Visipaque solution (iodixanol, GE Healthcare) and microCT images (Quantum FX; PerkinElmer, Waltham, USA) were taken at different timepoints (20 µm resolution, voltage 90 kV, current 180 mA, field of view = 10 mm). For each spheroid, the diameter was measured and a ROI of 0.1 x 1.8 mm was selected at the center of the spheroid. The changes in average pixel intensity within the ROI due to the inward diffusion of the contrast agent was monitored over time using the image processing software Image-J (Java, Redwood Shores, USA).

### Results

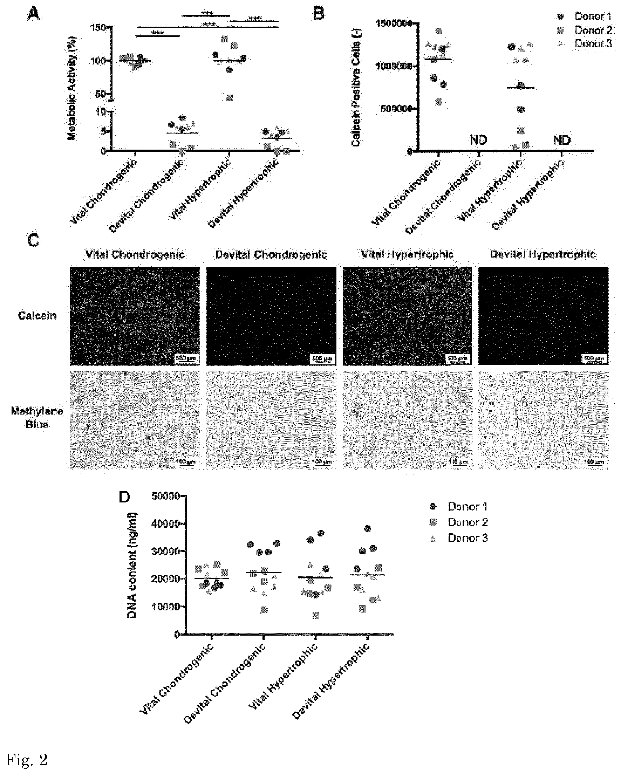

The results are illustrated in Figures 2-5.

Figure 2 shows cell viability after devitalization of engineered cartilage spheroids. (A) Metabolic activity before and after devitalization and (B) the number of living cells left as detected by calcein staining (green staining). (C) Calcein-positive cells extracted from the digested constructs were stained with methylene blue after replating and culturing for 2 days. *p<0.05, **p<0.01, *** p<0.001; ND: not detectable. Moreover, Figure 2D shows the DNA content of the spheroids after 31 day of culture. DNA content was not significantly affected by the culture medium (chondrogenic or hypertrophic) or the devitalization procedure. *p<0.05; **p<0.01; ***p<0.001.

The metabolic activity of the chondrogenic and hypertrophic samples was significantly reduced following the devitalization treatment, to 4.9±2.6 % and 3.7±2.3 % of their vital counterparts, respectively (Figure 2A). After digestion of the spheroids, no calcein-positive (living) cells were detected in the devitalized groups (Figure 2B). On the contrary, viable cells were observed for both vital chondrogenic and hypertrophic groups. Furthermore, no cells were attached to tissue culture plastic after the digested constructs were replated, while they were for the living controls (Figure 2C). Overall, no differences in viability between the chondrogenically differentiated samples and the samples stimulated into hypertrophy were observed. After devitalization, cellular debris was still present in the devitalized construct, as confirmed by the unchanged DNA content (Figure 2D).

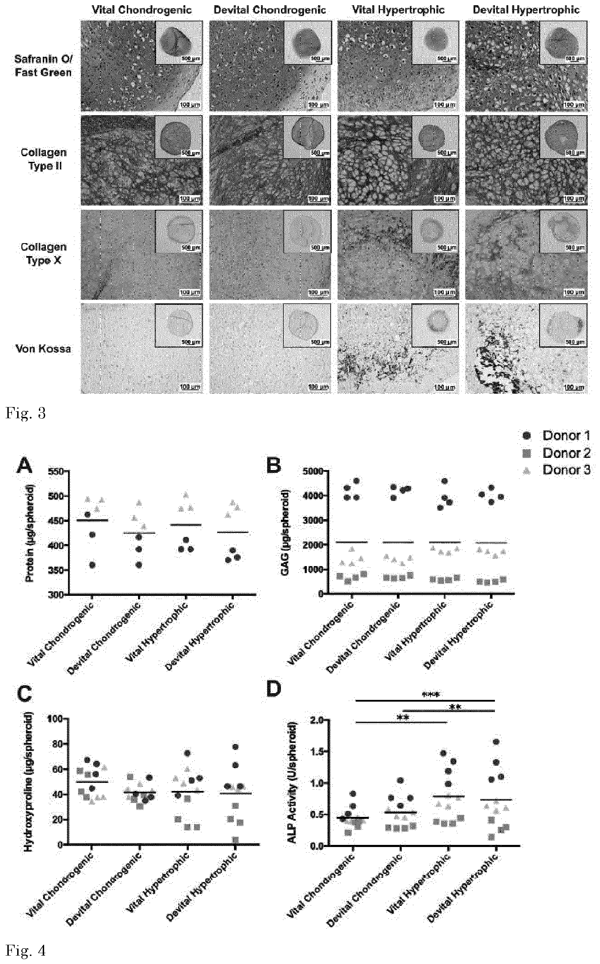

Figure 3 shows the qualitative evaluation of morphology and ECM preservation after devitalization. Sections of spheroids from the four experimental groups were stained for GAGs (red staining) in the top row, for collagen type II and X (brown staining) and for mineralization in the bottom row (dark brown). Inserts: appearance of complete spheroids. Figure 4 shows protein content and activity is retained in the cartilage constructs after devitalization. Quantification of (A) total protein content, (B) GAGs, and (C) total hydroxyproline in spheroids of the four groups before and after devitalization. (D) ALP activity was quantified as an indication of the retention of the bioactivity after devitalization. *p<0.05, **p<0.01, *** p<0.001.

ECM components were preserved after devitalization, as evaluated by histological analysis. There was no evident reduction in GAGs, collagen type II, collagen type X, and calcium content in the constructs (Figure 3). These observations were supported by the quantitative assays, where no statistically significant differences were found in total protein content (Figure 4A), GAG (Figure 4B) or hydroxyproline content (Figure 4C) between the devitalized and vital samples. Moreover, ALP activity, which is more likely to be affected by the devitalization than protein content, was not reduced after the devitalization procedure and was higher in the hypertrophic spheroids than in the chondrogenic ones (Figure 4D).

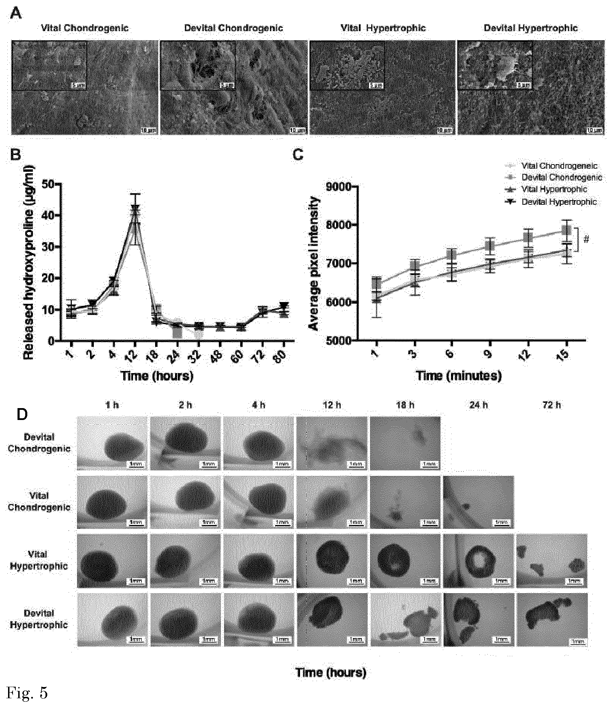

Figure 5 shows the ECM porosity and degradation rate. (A) SEM pictures highlight the presence of a more porous surface in the devitalized chondrogenic group compared to the others. (B) 2/3 devitalized chondrogenic spheroids were already degraded after 18 hours, whereas the last one was degraded 24 hours. The majority of the vital chondrogenic spheroids (2/3) was degraded by 24 hours, whereas 1/3 sample was degraded by 32 hours. A slower degradation rate was observed for the hypertrophic groups. (C) A higher average pixel intensity, which indicates higher amounts of iodixanol in the selected ROI, was observed in the devitalized chondrogenic group. IS: significant compared to devital hypertrophic; *p<0.05.

The surface roughness and porosity were evaluated by SEM analysis, highlighting the increased porosity at the surface of the chondrogenic devital samples, compared to the vital chondrogenic samples (Figure 5A). This difference was less evident in the hypertrophic group due to the minerals present on their surfaces. Furthermore, the increased porosity in devital chondrogenic samples was indirectly confirmed by their faster degradation rate compared to other groups (Figures 5B and 5D). In particular, all samples from the devitalized chondrogenic group were completely degraded after a maximum of 24 hours (degrading time 20 ± 3.5 h). This was 6.6 hours faster than their vital counterparts (degrading time 26.6 ± 4.6 h). For the hypertrophic group, the spheroids were not fully degraded after 80 hours, with no evident differences observed between the vital and devital groups. Consistently, the diffusion of iodixanol (Figure 5C), a neutrally charged contrast agent, into the selected ROI at the center of the spheroids, was highest in the devitalized chondrogenic group.

### Example 5 - Construct preparation for in vivo implantation

Chondrogenic differentiation and metabolic activity of the Dark Agouti MSCs was verified prior to *in vivo* implantation. For subcutaneous implantation, two chondrogenic spheroids per group (vital chondrogenic and hypertrophic; and devital chondrogenic and hypertrophic) were embedded in collagen (4 mg/mL) and cast in custom-made square cuboid molds (3 mm x 3 mm x 2 mm). Gelation was allowed for 45 minutes at 37°C according to manufacturer instructions. Empty collagen controls (n=6) were included as controls. For the orthotopic defects, eight chondrogenic spheroids were encapsulated in 3.5 mm x 3.5 mm x 6 mm custom-made molds in collagen gel, as described above. The constructs were prepared the day before implantation and incubated overnight in chondrogenic differentiation medium without TGF-β1 and BMP-2.

### Example 6 - Animal experiment and surgical procedures

The animal experiments were performed with the approval of the animal ethical committee of the University Medical Center Utrecht (2465-2-01) in accordance with the ARRIVE guidelines for animal experimentation [276]. Twenty-four male Brown Norway rats of 11 weeks old (Envigo) were randomly housed in pairs at the animal facility of the Utrecht University. Animals received standard food pellets and water ad libitum, under climate-controlled conditions (21 °C; 12 h light/12 h darkness). After 7 days of acclimatization, subcutaneous pockets were created from 5 mm dorsal incisions and blunt dissection as previously described (Gawlitta et al. Tissue Engineering Part A 21 (2015) 694-703) under general anaesthesia (1-3.5% isoflurane in oxygen, AST Farma, Oudewater, the Netherlands). In each pocket, one construct of either group (collagen control, vital chondrogenic, vital hypertrophic, devital chondrogenic, or devital hypertrophic) was implanted (n=6 per group). The skin was closed transcutaneously with Vicryl Rapide 4-0 sutures (VR 2297; Ethicon). Each animal received a maximum of 2 subcutaneous pockets. For implantation of the construct in a femur defect, a 6 mm critical-size segmental bone defect was created as previously described (Van der Stok et al. European Cells and Materials 27 (2014) 137-48; discussion 148) (n=8 for the devital chondrogenic and hypertrophic group and n=4 for the vital chondrogenic and hypertrophic controls). Briefly, the right hind leg was shaved and carefully disinfected. A lateral skin incision was made and soft tissue was dissected in order to expose the right femur. After the periosteum removal, three proximal and three distal screws were used to stabilize a 23 x 3 x 2 mm polyether ether ketone (PEEK) plate to the femur in the anterolateral plane. After fixation, a saw guide and a wire saw (RlSystem, Davos, Switzerland) were used to remove a 6 mm cortical bone segment. The collagen constructs were press-fit into the defect and a single dose of antibiotic (Duplocillin LA, 22.000 IE/kg, MSD Animal Health, Boxmeer, the Netherlands) was locally injected intramuscularly. The fascia and skin were sutured in layers using resorbable Vicryl Rapide 4-0 sutures (Ethicon). Subcutaneous injection of pain medication (buprenorphine, 0.05 mg/kg bodyweight, AST Farma, Oudewater, the Netherlands) was given pre-operatively and twice a day for the following three days. When devital constructs were implanted in the orthotopic defect, the rats also received only devital constructs subcutaneously.

### Example 7 - Analysis of bone regeneration

Following the experiment described in Example 6, rats were euthanized 12 weeks after surgery with an overdose of barbiturates (phenobarbital; 200 mg/kg body weight, TEVA Pharma, Haarlem, the Netherlands). The femora and the subcutaneous implants were retrieved and processed for histological analysis and micro-computed tomography (microCT) scanning.

### MicroCT scanning

Mineralization in the orthotopic defect area was assessed at 0, 4, 8, and 12 weeks after surgery. While under general anaesthesia, the hind leg of the rat was fixed to a custom-made support to allow scanning of the femur with a microCT imaging system (Quantum FX). Three minutes of scan time was required per leg for an isotropic voxel size of 42 µm resolution (voltage 90 kV, current 180 mA, field of view = 21 mm). All scans were oriented in the same fashion using the Image) plugin Reorient3 TP (Image-J 2.0.0; Java, Redwood Shores, CA, USA). A volume of interest (VOI) of 6.3 x 5 x 5 mm³ was selected. After euthanasia, subcutaneous implants were also analysed. Three minutes of scan time was required per subcutaneous implant for an isotropic voxel size of 20 µm resolution (voltage 90 kV, current 180 mA, field of view = 10 mm). After segmentation with a global threshold, the mineralized volumes (MV) for both the subcutaneous and femur implants were measured in mm³ using the image processing software plugin Bonet [277] (Image). 3D reconstructions of the femur defect were based on the microCT data and created using ParaView (ParaView 5.3.0, Kitware Inc, USA).

### Histological analysis of the in vivo samples

All specimens were fixed in a 10% neutral buffered formalin solution for 1 week and thereafter decalcified for 6 weeks in a 10% EDTA-phosphate buffered saline solution (pH 7.4). After decalcification, samples were additionally fixed for two days, dehydrated in a Leica ASP300S tissue processor in graded ethanol solutions (70-100%), cleared in xylene, embedded in paraffin and sliced into 5µm thick sections (Microm). Before staining, samples were deparaffinized with xylene and gradually rehydrated through decreasing ethanol solutions (100-70%). New bone formation was evaluated using H&E and Safranin/O fast green staining following the same staining procedure described in the previous section.

Histomorphometric analysis was performed for both the subcutaneous and orthotopic samples after H&E staining. Briefly, an overview of the whole sample was made by merging images into a panoramic image in Adobe Photoshop C6. For the subcutaneous implants, bone formation throughout the entire construct area was quantified. For the orthotopic implants, a region of interest (ROI) of 5 x 2.5 mm² was selected in the center of the defect. The titanium screw holes present on each side of the defect were used as reference point in order to ensure an equivalent positioning of ROI in all samples. Three different areas were manually selected for each ROI: bone, hypertrophic cartilage and bone marrow. The number of pixels for each area was quantified via the function "recording measurement" and expressed as a percentage of the total construct area for the ectopic implants and of the ROI area in the orthotopic ones.

### Results

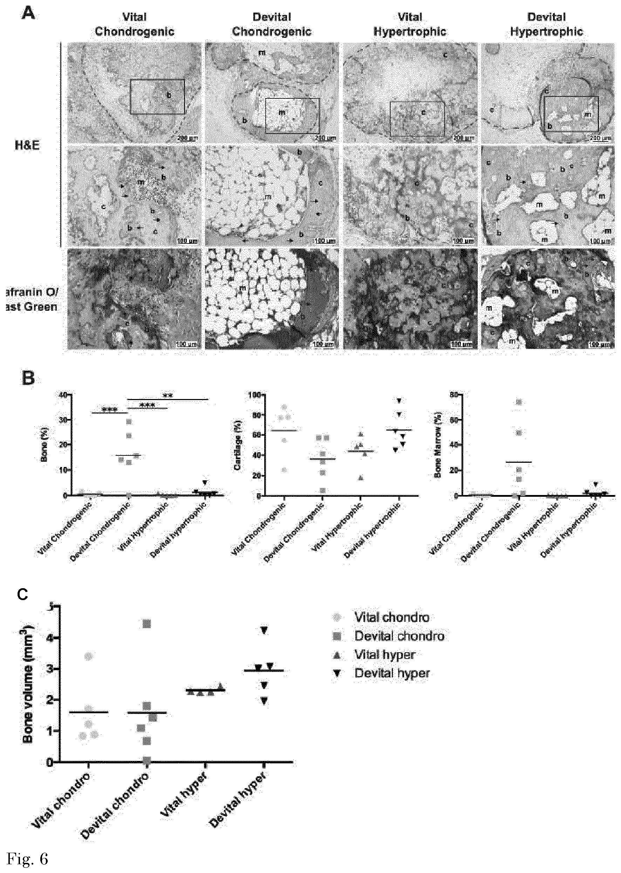

The results are illustrated in Figures 6-9.

Figure 6 shows the subcutaneous endochondral bone formation 12 weeks post implantation. (A) Overview of the implanted constructs stained with H&E. The grey dotted lines indicate the edges of the constructs whereas the black boxes highlight the area depicted in the higher magnification pictures. New bone formation was observed in at least a few samples of all groups (bright pink in the H&E). Furthermore, bone marrow was observed, especially in both devitalized groups. Non-remodelled cartilage was evident in all groups (red staining in the Safranin O/fast green). b: bone; c: cartilage; m: bone marrow; arrows: osteocytes. (B) Results of the histomorphometric analysis performed 12 weeks post implantation display the highest bone formation for the devital chondrogenic group. *p<0.05; **p<0.01; ***p<0.001.

No difference in mineralized tissue volume was observed in the subcutaneous implants, with roughly 1-3 mm³ mineralized tissue volume present in all groups (Figure 6C). In contrast, differences in new bone formation were observed based on histological evaluation (Fig. 6A,B). In more detail, small, localized areas of bone formation were observed in the vital chondrogenic, vital hypertrophic and devital hypertrophic group (0.48 ± 0.68%, 0.23 ± 0.33% and 1.2 ± 1.8% of the total construct area, respectively). Nevertheless, a larger amount of neo-bone formation was observed in the devital chondrogenic group, with an average of 16.5 ± 11% of the total construct area. Although not statistically significant, on average larger areas of bone marrow were also observed in this group (26 ± 29%) compared to the vital chondrogenic (0.12 ± 0.27%), vital hypertrophic (0.1 ± 0.17%) and devital hypertrophic (2.1 ± 3.4%) ones. Finally, remnants of areas rich in GAGs were observed in all groups.

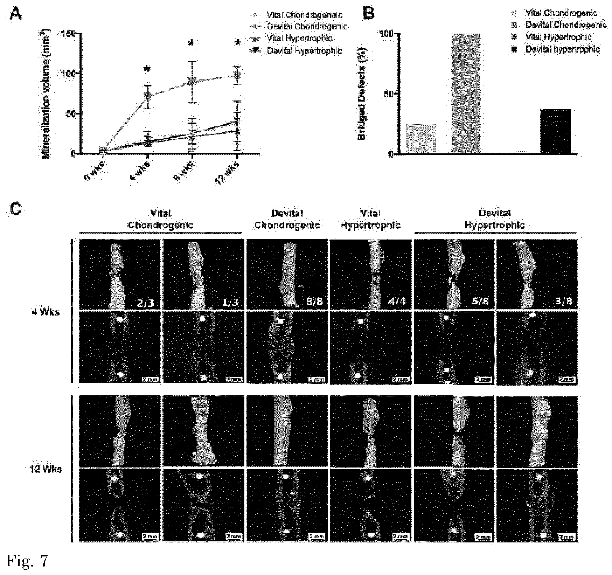

Figure 7 shows micro-CT-based evaluation of bone formation in the femur defects. (A) Quantification of mineralization over time for all groups. (*p<0.05) (B) Different rate of defect bridging were observed between groups. Full defect healing was observed in 8/8 samples of the devital chondrogenic group (100%), 3/8 samples of the hypertrophic devital group (37.5%), 1/3 sample of the chondrogenic vital control group (33%) and in none of the vital hypertrophic control group. (C) 3D reconstructions and 2D images of the defects 4 and 12 weeks post-implantation. White circles are titanium screws.

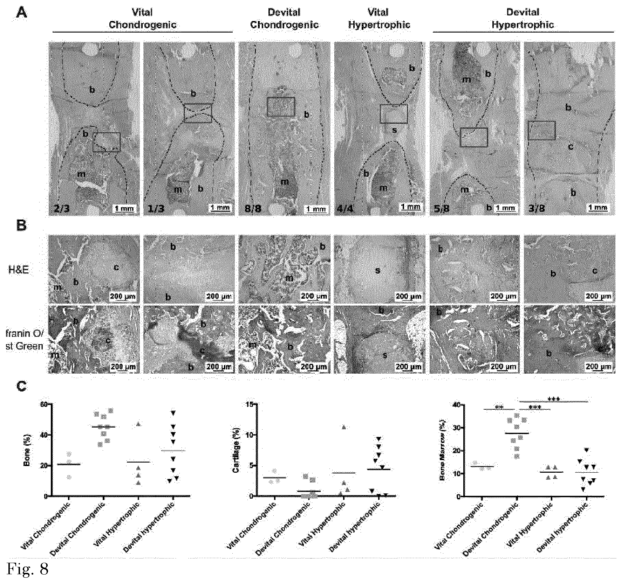

Figure 8 shows endochondral bone formation in the femur defects 12 weeks post implantation. (A) Overview of the femur defects. The black dotted lines indicate the bone edges whereas the grey boxes highlight the magnified areas depicted in B. (B) Higher magnification images of H&E and Safranin 0/fast green staining. New bone formation was observed at the defect edges for all groups. Remodelling and formation of a distinct cortical and cancellous compartment was observed in all samples of the devital chondrogenic group. Non-remodelled spheroids were still present in the center of the defect of a few samples belonging to both vital groups and the devital hypertrophic group. (C) In the histomorphometric analysis presented a trend in bone formation comparable to the mineralization observed by microCT is evident. *p<0.05; **p<0.01; ***p<0.001. b: bone; c: cartilage; s: spheroid; m: bone marrow.

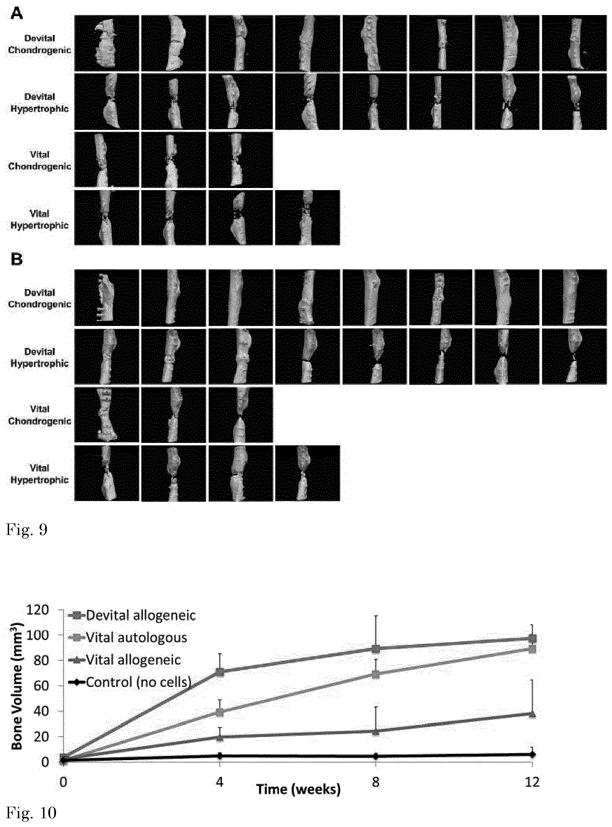

Figure 9 shows 3D reconstructions of the femur defects. (A) 3D reconstructions after 4 weeks and (B) 12 weeks.

New mineralized bone formation was observed in all groups based on the microCT data and histological analysis (Figures 7 and 8). The devitalized chondrogenic group outperformed all other groups, with a final mineralization volume of 97.4 mm³ ± 11.2 after 12 weeks. The mineralization volume of the devitalized hypertrophic group was 40.6 mm³ ± 25.7, 38 mm³ ± 26.6 for the vital chondrogenic group, and 28.2 mm³ ± 23.9 for the vital hypertrophic group (Figure 7A). In addition, a statistically significant increase in mineralization volume and complete mineralization of the defects already occurred after 4 weeks for the devitalized chondrogenic group (Figures 7A, 7C and Figure 9). After 12 weeks full healing was observed in all 8 samples (100%) of the chondrogenic devital group. On the contrary, varying results were obtained in the other groups, with full bridging in 3/8 samples of the hypertrophic devital group (37.5%), in 1/3 samples of the chondrogenic vital control group (33%), and in none of the samples of the vital hypertrophic control group (0%) (Figure 7B).

H&E staining showed that in the chondrogenic devital group, the newly formed bone was remodelled and both the cortical and trabecular compartment of the femur was restored after 12 weeks. In only 2/8 samples, small areas characterized by the presence of hypertrophic chondrocytes (3% of the ROI) were still observed after 12 weeks, indicating ongoing remodelling at the proximal edge of the defect (Figure 8). On the contrary, no cortical and trabecular compartments could be identified at the defect center of the other groups, not even in the fully bridged samples (Figures 7C, 8A and 8B). Traces of the non-remodelled cartilaginous spheroids were found in the vital groups and the hypertrophic devital group (Figure 8).

### Comparative Example 1

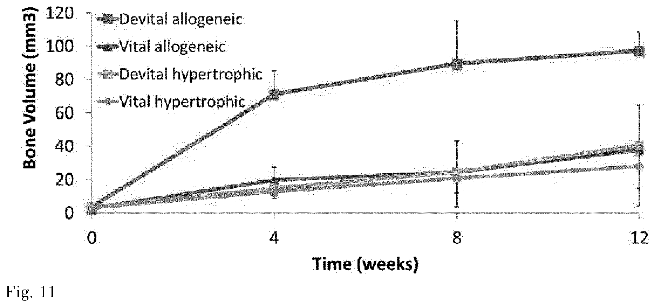

Endochondral bone formation using vital constructs (both chondrogenic and hypertrophic) as prepared in Example 2 was analyzed in a model as described in Example 7. In addition, endochondral bone formation using constructs based on chondrogenically differentiated, non-autologous (both allogeneic and xenogeneic) mesenchymal stromal cells (MSCs) embedded in a collagen carrier as described in Longoni et al. Frontiers in Bioengineering and Biotechnology, 8 (2020) Article 651, is tested and analyzed in a model as described in Examples 6 and 7. The results are illustrated in Figures 10 and 11 to compare them to the constructs (both chondrogenic and hypertrophic) obtained according to Example 3.

Figure 10 shows the increase of bone volume over time after implantation of vital autologous engineered cartilage tissue (Longoni *et al.* 2020), vital allogeneic engineered cartilage tissue (Example 2) and devital allogeneic engineered cartilage tissue according to the invention (Example 3). A control with no cells and only collagen is included as well (Longoni *et al.* 2020). Figure 9 shows that the devital allogeneic construct according to the invention most effectively promotes bone generation.

Figure 11 shows the increase of bone volume in time after implantation of vital chondrogenic and hypertrophic allogeneic constructs (Example 2) and of devital chondrogenic and hypertrophic allogeneic constructs (Example 3). The devitalized constructs more effectively promote bone generation than their vital equivalents, while the chondrogenic devital allogeneic construct according to the invention most effectively promotes bone generation.

## Claims

1. Method for providing a devitalized engineered cartilaginous tissue that is suitable for bone regeneration, said method comprising the steps of:
- providing cells, preferably encapsulated in a matrix, wherein the cells are chondrogenic or are capable of giving rise to cells to the chondrogenic lineage;
- differentiating said cells into cartilage cells comprising chondrocytes, chondrocytes expressing one or more hypertrophic markers and/or hypertrophic chondrocytes to obtain a vital cartilaginous tissue;
- mildly devitalizing the vital cartilaginous tissue comprising drying, preferably lyophilizing, to obtain the engineered devitalized cartilaginous tissue.

2. Method according to claim 1, wherein the cells are selected from the group consisting of pluripotent stem cells such as embryonic stem cells, induced pluripotent stem cells (iPSCs), cells of the mesoderm lineage, cells of the mesenchymal lineage including mesenchymal stem cells and multipotent mesenchymal stromal cells such as multipotent mesenchymal stromal cells isolated from fat (stromal vascular fraction or adipose-derived stem cells), bone marrow, cartilage, teeth, blood, umbilical cord, placenta, Wharton's jelly, bone (skeletal stem cells), skeletal muscle (muscle-derived stem cells or satellite cells) or other tissues or organs, and combinations thereof.

3. Method according to any of the previous claims, wherein the cells are non-autologous and preferably allogeneic.

4. Method according to any of the previous claims, wherein said cells are chondrogenically differentiated, preferably wherein differentiating said cells comprises contacting the cells with one or more members of the transforming growth factor-B (TGF-β) superfamily, in particular TGF-β isoforms and/or bone morphogenetic proteins (BMPs).

5. Method according to any of the previous claims, wherein the cells are encapsulated in a matrix and said matrix comprises a natural, semi-synthetic or fully synthetic matrix, preferably comprising one or more materials of natural hydrogels, semi-synthetic or fully synthetic hydrogels, polymers, biomaterials, implantable metals and/or calcium phosphates.

6. Devitalized engineered cartilaginous tissue for bone regeneration obtainable in a method in accordance with any of the previous claims.

7. Devitalized engineered cartilaginous tissue according to the previous claim, being maximally 5 mm, preferably 3 mm, more preferably 2 mm, in at least one direction and preferably having the shape of a spheroid, cylinder, block, sheet, rod, tube or the like.

8. Devitalized engineered cartilaginous tissue in accordance with any of claims 6-7, wherein said tissue comprises pores, preferably pores having a size of more than 0.1 µm, more preferably with pores having a size in the range of 0.1 to 50 µm such as 1 to 10 µm.

9. Devitalized engineered cartilaginous tissue in accordance with any of claims 6-8, exhibiting a metabolic activity of less than 50%, preferably less than 10%, most preferably less than 5%, compared to the metabolic activity of equivalent vital cartilaginous tissue comprising cartilage cells.

10. Devitalized engineered cartilaginous tissue in accordance with any of claims 6-9, comprising extracellular matrix (ECM) components, preferably at least glycosaminoglycans (GAGs), collagen type II, collagen type X and/or calcium.

11. Devitalized engineered cartilaginous tissue in accordance with any of claims 6-10, comprising cellular debris and preferably exhibiting a total DNA content, glycosaminoglycan (GAG), total protein content and/or an alkaline phosphatase (ALP) activity that is no less than 50%, preferably no less than 75%, most preferably no less than 90% compared to the total DNA content, GAG, total protein content and/or an ALP activity of equivalent vital cartilaginous tissue comprising cartilage cells.

12. Devitalized engineered cartilaginous construct comprising a plurality of the devitalized engineered cartilaginous tissue in accordance with any of claims 6-11 immobilized in or on a carrier, preferably wherein the carrier comprises a natural, semi-synthetic or fully synthetic implantable material, more preferably one or more of natural, semi-synthetic or fully synthetic hydrogels, polymers, biomaterials, metals and/or calcium phosphates.

13. Method for preparing the devitalized engineered cartilaginous construct according to the previous claim, comprising providing the devitalized engineered cartilaginous tissue in accordance with any of claims 6-11 and the carrier and immobilizing the devitalized engineered cartilaginous tissue in or on the carrier, wherein the method optionally comprises pulverizing or otherwise pretreating the devitalized engineered cartilaginous tissue before immobilization.

14. Kit of parts comprising the devitalized engineered cartilaginous tissue according to any of claim 6-11 and a carrier, preferably wherein the carrier comprises a natural, semi-synthetic or fully synthetic implantable material, more preferably one or more of natural, semi-synthetic or fully synthetic hydrogels, polymers, biomaterials, metals and/or calcium phosphates.

15. Devitalized engineered cartilaginous tissue or the devitalized engineered cartilaginous construct according to any of claims 6-13 for use in a medical treatment, preferably for use in a medical treatment comprising tissue regeneration, more preferably for use in a medical treatment comprising bone regeneration, most preferably wherein said use comprises administering said bone regeneration construct or bone regeneration composition at a bone defect or augmentation site, preferably due to congenital, developmental or acquired bone defects such as a maxillofacial, neurosurgical or orthopedic bone defect, including segmental mandibular, or maxilla bone defect or skull defects or long bone defects, hand defects, non-unions, or bone resection sites or wherein said use comprises administering said bone regeneration construct or bone regeneration composition to augment existing bone tissue, such as for example required for spinal fusions, sinus floor augmentation, alveolar augmentation of mandible or maxilla for dental reconstructions with or without dental implants.

16. Devitalized cartilaginous tissue or the devitalized engineered cartilaginous construct for use according to claim 15, wherein the devitalized cartilage tissue comprises non-autologous and preferably allogeneic devitalized cartilage tissue.
